# EUROPEAN PATENT SPECIFICATION

(11) **EP 1 908 486 B1**
(45) Date of publication and mention of the grant of the patent: **21.03.2012**
(21) Application number: 06746616.9
(22) Date of filing: 18.05.2006
(51) Int. Cl.: A61M 1/14, A61M 1/16, A61M 1/36

(54) **APPARATUS FOR DIALYTIC THERAPY**
DIALYSETHERAPIEGERÄT
APPAREIL DE TRAITEMENT DIALYTIQUE

(30) Priority: 22.06.2005 JP 2005181721
(43) Date of publication of application: 09.04.2008
(73) Proprietor: NIKKISO COMPANY, LTD., Shibuya-ku, Tokyo 150-8677 (JP)
(72) Inventor: SUGIOKA, Akira, Nikkiso Company Limited, Makinohara-shi, Shizuoka 421-0496 (JP); MORI, Yoshihiro, Nikkiso Company Limited, Makinohara-shi, Shizuoka 421-0496 (JP); TOYODA, Masahiro, Nikkiso Company Limited, Makinohara-shi, Shizuoka 421-0496 (JP)
(74) Representative: GROSSE SCHUMACHER KNAUER VON HIRSCHHAUSEN
(86) International application number: PCT/JP2006/309941
(87) International publication number: WO 2006/137228

(56) References cited:
- EP-A1- 1 364 666
- EP-A2- 0 575 712
- WO-A1-02/053209
- WO-A2-2004/074966
- JP-A- 2004 248 793
- US-A- 5 609 770

## Description

### TECHNICAL FIELD

The present invention relates to a hemodialysis treatment apparatus for carrying out a hemodialysis treatment by performing hemodialysis and ultrafiltration upon an extracorporeally-circulating blood of a patient.

### BACKGROUND ART

Generally, a hemodialysis treatment uses a hemodialysis apparatus that includes a blood circuit for extracorporeally circulating blood of a patient, a dialyzer provided at an intermediate portion of the blood circuit, a peristaltic type blood pump, and a dialysis device body for performing hemodialysis treatment by inletting and outletting dialysate from and to the dialyzer while ultrafiltrating. Note that, the blood circuit primarily includes an arterial blood circuit having an arterial needle at end thereof, and a venous blood circuit having a venous needle at an end thereof.

When the arterial needle and the venous needle are inserted into a patient and the blood pump is driven, blood of the patient is introduced into the blood circuit through the arterial needle, and then returns into the body of the patient through the arterial blood circuit, the dialyzer and the venous blood circuit. The dialyzer includes a plurality of hollow fibers forming hemodialysis membranes. The blood flows inside of the hollow fibers, and the dialysate with a predetermined concentration supplied from the dialysis device body flows outside (i.e., between the outer surfaces of the hollow fibers and the inner surface of the body of the dialyzer) of the hollow fibers so that waste products within the blood flowing through the inside of the hollow fibers permeate into the dialysate through the hemodialysis membrane.

Accordingly, the blood from which waste products are removed returns into the body of the patient through the venous blood circuit. On the other hand, the dialysis device body is provided with an ultrafiltration pump for removing surplus water from the blood of a patient so that the blood is ultrafiltrated through the hemodialysis membranes during the hemodialysis treatment. A volume of water (ultrafiltration rate) to be ultrafiltrated by the ultrafiltration pump is adjusted by controlling the driving of the ultrafiltration pump.

When a volume of water to be ultrafiltrated (ultrafiltration volume) is large, it is required to increase the ultrafiltration rate, so that the patient may show a shock symptom due to a low blood pressure and the like, depending on health conditions of the patient. In order to catch an omen of the shock symptom, a monitoring apparatus has been proposed for monitoring conditions of a patient during the hemodialysis treatment by detecting a hematocrit value (a volume ratio of red blood cells to the whole blood) of the blood of the patient to calculate a variation rate of a volume of circulating blood (ΔBV) of the patient by using the hematocrit value.

That is, although the circulating blood volume variation rate (ΔBV) of a patient generally decreases due to ultrafiltration as the treatment time passes, a sudden decrease in ΔBV is considered to be an omen of shock symptoms due to a low blood pressure and the like, so that it is considered that performing a proper preventive treatment (additionally supplying of saline, or suspending of the hemodialysis treatment) upon the patient at the time of the sudden decrease in ΔBV may prevent the shock symptoms. Thus, a hemodialysis treatment apparatus capable of successively measuring a hematocrit value of a patient during the hemodialysis treatment to detect a circulating blood volume variation rate (ΔBV) of the patient by using the hematocrit value is disclosed in patent document JP-2004-97781-A, for example.

EP 1 364 666 A1 discloses a biological information and blood treating device information control system with all features of the preamble of claim 1.

WO 2004/074966 A2 relates to a bed-side information system. The information system comprises a body information monitor which measures i.a. the arterial blood pressure of the patient. A hematocrit monitor is also provided. The information detected by the sensors is sent to a desktop PC for evaluation. The desktop PC displays the arterial blood pressure and the hematocrit value and other related blood values on a display.

### SUMMARY OF THE INVENTION

### PROBLEMS TO BE SOLVED BY THE INVENTION

However, in the above described prior art hemodialysis treatment apparatus, although a circulating blood volume variation rate (ΔBV) of a patient is monitored to catch an omen of shock symptoms to a certain degree, there was a problem in that it is difficult to compare ΔBV with vital signs (bio-information of the patient during the hemodialysis treatment, such as a blood pressure and the pulse). That is, since a sudden change in ΔBV is not always an omen of shock symptoms, also it may occur due to external factors such as changes in hemodialysis conditions including changes in the ultrafiltration rate, changes in body positions from a lying state to a sitting-up state and changes in taking food and medicines, it is difficult to determine whether or not a sudden change of ΔBV is an omen of shock symptoms.

Note that, a hemodialysis treatment apparatus for successively detecting and displaying a vital sign (such as a blood pressure) aside from ΔBV has been proposed, so that it is conceivable that the origin of a sudden decrease of ΔBV can be determined by displaying ΔBV and the vital sign in respective display means (ΔBV and the vital sign may be switched and displayed in separate windows of the identical display means) to compare their trends. In that case, however, since it is required to compare data by turning his/her eyes to respective display screens (respective windows switched and displayed in the identical display means), which is troublesome in monitoring for medical staffs, and since it is difficult to compare parameters from each other, there was a problem in that it is difficult to determine whether or not the sudden decrease of ΔBV is an omen of a shock symptom.

In view of the foregoing circumstances, the present invention is intended to provide a hemodialysis treatment apparatus, which can easily compare a circulating blood volume variation rate (ΔBV) with a vital sign when the circulating blood volume variation rate (ΔBV) of a patient is suddenly decreased, and easily determine whether or not the sudden decrease is an omen of shock symptoms during the hemodialysis treatment.

### MEANS FOR SOLVING THE PROBLEM

The invention described in claim 1 is a hemodialysis treatment apparatus for carrying out a hemodialysis treatment by performing hemodialysis and ultrafiltration upon an extracorporeally-circulating blood of a patient, which is characterized by comprising a circulating blood volume variation rate detecting means for successively detecting a variation rate of a volume of circulating blood of the patient as a hemodialysis treatment time passes, a vital sign detecting means for successively detecting a vital sign of the patient as a hemodialysis treatment time passes, and a display means capable of displaying a value detected by the circulating blood volume variation rate detecting means and a value detected by the vital sign detecting means on a time basis on an identical screen.

The hemodialysis apparatus is provided with a storage means for storing values detected on a time basis by the circulating blood volume variation rate detecting means in past hemodialysis treatments, the display means displaying past detected values stored in the storage means on the identical screen on a time basis in addition to the values detected by the circulating blood volume variation rate detecting means and the vital sign detecting means in the hemodialysis treatment.

The invention described in claim 2 is the hemodialysis treatment apparatus as set forth in claim 1, which is characterized by comprising a hemodialysis condition detecting means for successively detecting a hemodialysis condition relating to hemodialysis and ultrafiltration as the hemodialysis treatment time passes, the display means displaying a value detected by the hemodialysis condition detecting means in addition to the value detected by the circulating blood volume variation rate detecting means and the value detected by the vital sign detecting means on a time basis on the identical screen.

The invention described in claim 3 is the hemodialysis treatment apparatus as set forth in claim 1 or claim 2, which is characterized in that the display means displays each of successively-detected values in graphs each with an abscissa designating the time.

The invention described in claim 4 is the hemodialysis treatment apparatus as set forth in claim 3, which is characterized in that the display means displays each of the successively-detected values in a plurality of graphs overlapping with each other and with a common time axis.

The invention described in claim 5 is the hemodialysis treatment apparatus as set forth in claim 4, which is characterized in that the display means displays only a graph designating a detected value arbitrarily selected among the graphs being displayed and overlapping with each other.

The invention described in claim 6 is the hemodialysis treatment apparatus as set forth in any one of claim 1 to claim 5, which is characterized in that the display means displays an external factor that has an influence on the hemodialysis treatment for the patient and a duration where the external factor has influenced, so that the detected values are compared with each other on a time basis.

The invention described in claim 7 is the hemodialysis treatment apparatus as set forth in claim 1, which is characterized in that an ideal range of transitions is obtained for the values detected by the circulating blood volume variation rate detecting means based on the past detected values stored in the storage means, so that an upper limited value and a lower limited value of the ideal range of transitions are displayed on a time basis on the identical screen.

### EFFECT OF THE INVENTION

According to the invention of claim 1, since the values successively detected by the circulating blood volume variation rate detecting means and the values successively detected by the vital sign detecting means are displayed on a time basis on the identical screen, when a circulating blood volume variation rate (ΔBV) of a patient suddenly decreases during a hemodialysis treatment, the above-mentioned ΔBV can be easily compared with vital signs, thus easily determining whether or not the sudden decrease is an omen of shock symptoms.

In addition to the values detected by the circulating blood volume variation rate detecting means and the vital sign detecting means in the current hemodialysis treatment, since the past detected values stored in the storage means are displayed on the identical display means on a time basis, a tendency of the past variation rate of circulating blood volume can be easily compared with a tendency of the current variation rate of circulating blood volume, thus performing an optimal hemodialysis treatment.

According to the invention of claim 2, in addition to the values detected by the circulating blood volume variation rate detecting means and the values detected by the vital sign detecting means, since the values detected by the hemodialysis condition detecting means can be displayed on a time basis on the identical screen, when the circulating blood volume variation rate (ΔBV) of a patient suddenly decreases, it can be easily determined whether or not the sudden decrease in ΔBV is due to changes in the hemodialysis conditions.

According to the invention of claim 3, since each of successively-detected values are displayed in graphs each with an abscissa designating the time, a tendency (trend) of each of the detected values can be perceived, thus easily comparing the detected values with each other.

According to the invention of claim 4, since each of successively-detected values are displayed on the common time axis in graphs overlapping with each other, a tendency (trend) of each of the detected values can be perceived and a comparison of those detected values can be visually performed, thus easily identifying a cause when a circulating blood volume variation rate (ΔBV) suddenly decreased.

According to the invention of claim 5, since only a graph designating a detected value arbitrarily selected from graphs being displayed and overlapping with each other is displayed, graphs displayed on the display means are improved in the visibility, thus easily and properly comparing tendencies (trends) of required detected values with each other.

According to the invention of claim 6, since the external factor that has an influence on the hemodialysis treatment for a patient and the duration for the external factor has influenced can be displayed and compared with detected values displayed on a time basis, when the circulating blood volume variation rate (ΔBV) of a patient suddenly decreased, it is possible to easily determine whether or not the sudden decrease in ΔBV is due to external factors.

According to the invention of claim 7, when an ideal range of transition of detected values is obtained based on the past values detected by the circulating blood volume detecting means, since the upper limited value and the lower limited value of the ideal range of transition are displayed on a time basis on the identical screen, it is possible to monitor in real-time whether or not the current value detected by the circulating blood volume detecting means is within the ideal range of transition, and to easily compare the current value with the value detected by the vital sign detecting means when the current value deviates from the ideal range of transition.

### BEST MODE OF CARRYING OUT THE INVENTION

Hereinafter, a mode of carrying out the present invention will be explained in detail with reference to the accompanying drawings.

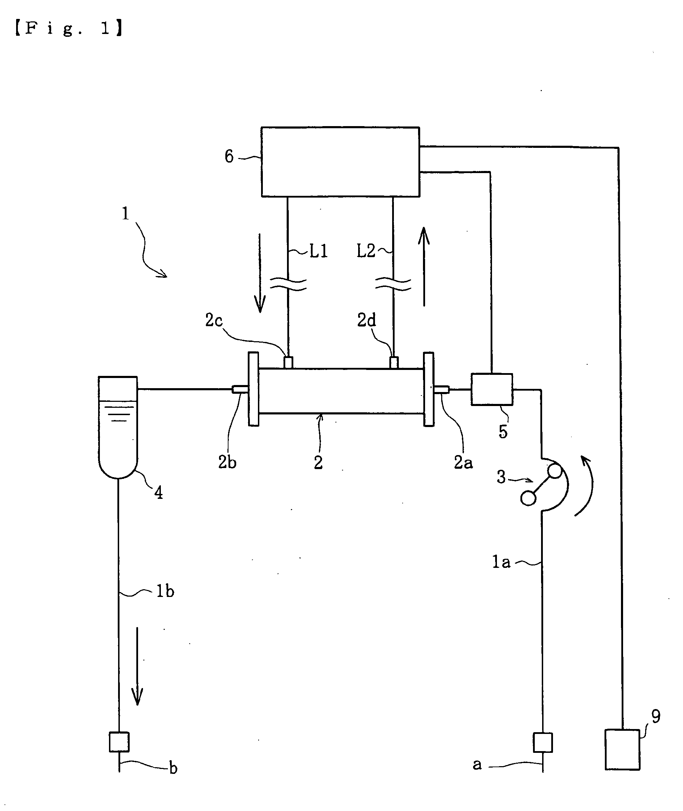

As shown in Fig. 1, a hemodialysis treatment apparatus according to the first carrying-out mode is used to perform hemodialysis and ultrafiltration by extracorporeally circulating blood of a patient and is mainly constructed by a blood circuit 1 for extracorporeally circulating the blood of the patient, a dialyzer 2 connected to the blood circuit 1 for a hemodialysis treatment, and a dialysis device body 6 connected to the dialyzer 2 for ultrafiltration of the blood while supplying dialysate. As shown in Fig. 1, the blood circuit 1 is mainly constructed by an arterial blood circuit 1a and a venous blood circuit 1b each formed by flexible tube. The dialyzer 2 is connected between the arterial blood circuit 1 a and the venous blood circuit 1 b.

The arterial blood circuit 1 a is provided with an arterial needle "a" at an end portion of the arterial blood circuit, and also provided with a roller-type blood pump 3 and a hematocrit sensor 5 in the middle of the arterial blood circuit. The venous blood circuit 1 b is provided with a venous needle "b" at an end portion thereof, and a drip chamber 4 for removing bubbles in the middle thereof.

The hematocrit sensor 5 is provided with a light emitting element such as an LED and a light receiving element such as a photodiode. The hematocrit sensor 5 irradiates the blood with a light of a predetermined wavelength from the light emitting element, and receives a transmitted or reflected light by the light receiving element to measure a hematocrit value indicating a concentration of the blood flowing through the blood circuit 1. That is, the hematocrit value is a benchmark indicating a concentration of blood, and is expressed by a ratio of a volume of red blood cells to a volume of whole blood, in more concrete terms.

When the blood pump 3 is driven while the arterial needle "a" and the venous needle "b" are inserted into the patient, the blood of the patient flows through the arterial blood circuit 1 a into the dialyzer 2 where the blood is purified, and flows into the drip chamber 4 where bubbles within the blood are removed, and then returns to the body of the patient through the venous blood circuit 1b. Thus, the blood of the patient is dialyzed by the dialyzer 2 during an extracorporeal circulation through the blood circuit 1.

The dialyzer 2 is provided with a blood inlet port 2a, a blood outlet port 2b, a dialysate inlet port 2c and a dialysate outlet port 2d at a body thereof. The blood inlet port 2a and the blood outlet port 2b are each connected to ends of the arterial blood circuit 1a and the venous blood circuit 1b, respectively. Additionally, the dialysate inlet port 2c and the dialysate outlet port 2d are connected to a dialysate inlet line L1 and a dialysate outlet line L2, respectively, both extending from the dialysis device body 6.

The dialyzer 2 includes a plurality of hollow fibers. The blood flows through the inside of the hollow fibers, and the dialysate flows through a portion between the outer surfaces of the hollow fibers and the inner surface of the body member of the dialyzer 2. In the hollow fibers, a large number of micro holes (pores) penetrating between the inner and outer surfaces of the hollow fibers are formed to form permeable membranes where impurities and the like in the blood are permeated into the dialysate.

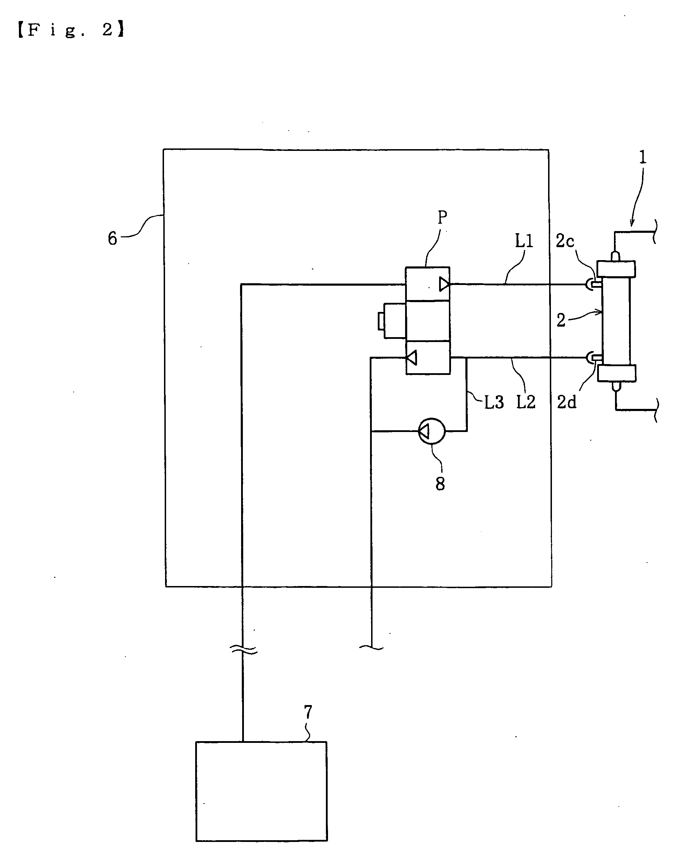

As shown in Fig. 2, the dialysis device body 6 is mainly constructed by a duplex pump P bridging the dialysate inlet line L1 and the dialysate outlet line L2, a bypass line L3 bypassing the duplex pump P in the dialysate outlet line L2, and an ultrafiltration pump 8 connected to the bypass line L3. One end of the dialysate inlet line L1 is connected to the dialyzer 2 (the dialysate inlet port 2c), and another end is connected to a dialysate supplying device 7 for producing a predetermined concentration of dialysate.

One end of the dialysate outlet line L2 is connected to the dialyzer 2 (the dialysate outlet port 2d), and another end is connected to a fluid disposal means (not shown) so that the dialysate supplied from the dialysate supplying device 7 flows through the dialysate inlet line L1 into the dialyzer 2 and then flows through the dialysate outlet line L2 and the bypass line L3 into the fluid disposal means.

The ultrafiltration pump 8 is used for removing water from the blood of a patient flowing through the dialyzer 2. When the ultrafiltration pump 8 is driven, the duplex pump P is of a quantitative type, so that a volume of the dialysate flowing through the dialysate outlet line L2 from the dialyzer becomes greater than that of the dialysate flowing through the dialysate inlet line L1 into the dialyzer. Accordingly, the amount of water corresponding to the difference between the past volume of the dialysate and the latter volume of the dialysate is removed from the blood. Note that, devices other than the ultrafiltration pump 8 (a so-called balancing chamber, for example) may be used for removing water from the blood of a patient. Note that, the duplex pump P and the ultrafiltration pump 8 form a dialysis treatment means for performing hemodialysis treatment and ultrafiltration for an extracorporeally-circulated blood of a patient.

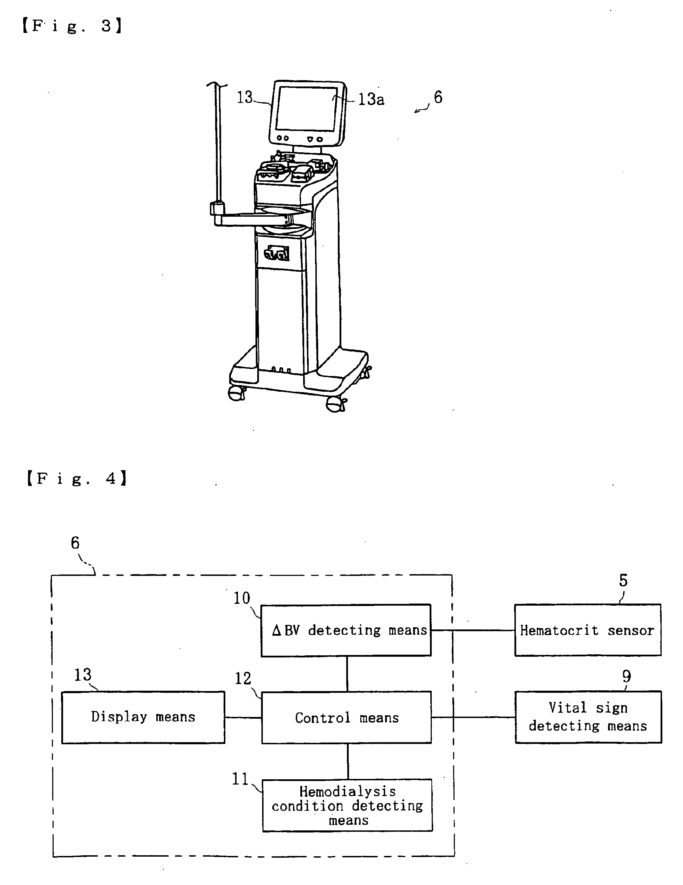

Now, the dialysis device body 6 according to the present carrying-out mode is electrically connected to a vital sign detecting means 9. The vital sign detecting means 9 is attached on a patient during the hemodialysis treatment, and is constructed to successively detect vital signs such as a blood pressure and the pulse of the patient as the time of the hemodialysis treatment passes and to output detected values to the dialysis device body 6 (specifically, a control means 12 shown in Fig. 4). The vital signs are bio-information of a patient under the hemodialysis treatment, i.e., omens representing the living of a living organism such as a breath, a body temperature, oxygen saturation and a perspiration rate in addition to the blood pressure and the pulse of a patient described above.

Also, as shown in Fig. 3, the dialysis device body 6 is provided with a display means 13 for displaying the current state of the hemodialysis treatment and the like (such as the time passed from the beginning of the treatment, an ultrafiltration rate) on a touch panel type of liquid crystal screen 13a, for example. As shown in Fig. 4, the display means 13 is electrically connected to a control means 12, and the control means 12 is electrically connected to a circulating blood volume variation rate detecting means 10 (hereinafter, referred to as a ΔBV detecting means) and to a hemodialysis condition detecting means 11.

The ΔBV detecting means 10 is electrically connected to the aforementioned hematocrit sensor 5, and is used for calculating and detecting a circulating blood volume variation rate (ΔBV) based on a hematocrit value transmitted from the hematocrit sensor 5. Incidentally, when a hematocrit value obtained by the hematocrit sensor 5 is defined as Ht, a circulating blood volume variation rate ΔBV can be obtained by the formula of (Ht at the starting time of the hemodialysis treatment - Ht at the time of measurement) / (Ht at the time of measurement) x 100. This can successively detect the circulating blood volume variation rate (ΔBV) as the time of the hemodialysis treatment passes.

The hemodialysis condition detecting means 11 is used for successively detecting hemodialysis conditions (such as an ultrafiltration rate, and a venous blood pressure obtained by detecting the internal pressure of an air-layer side of the venous drip chamber 4 in the venous blood circuit 1b) relating to hemodialysis and ultrafiltration as the time of the hemodialysis treatment passes. All values detected by the hemodialysis condition detecting means 11, the aforementioned ΔBV detecting means 10 and the vital sign detecting means 9 are transmitted to the control means 12 which performs a predetermined processing, and are displayed by the display means 13.

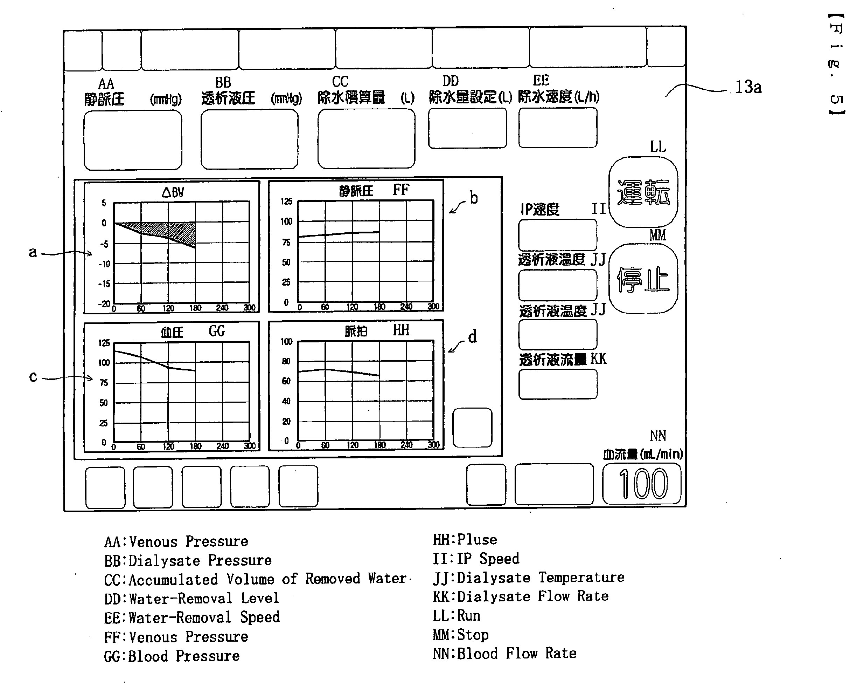

In more concrete terms, the control means 12 is used for controlling the values detected by each of the detecting means (the vital sign detecting means 9, the ΔBV detecting means 10 and the hemodialysis condition detecting means 11) so that the detected values are displayed in parallel on the identical screen on a time basis. Thus the screen 13a of the display means 13 is displayed as shown in Fig. 5, for example. In Fig. 5, graphs "a" to "d" with the abscissa designating the time (time passed from the beginning of the current (present) hemodialysis treatment) are displayed in the identical screen13a. Note that, on the screen 13a, a variety of information (such as dialysate pressure, an accumulated ultrafiltration volume and dialysate temperature) in the current hemodialysis treatment is displayed by numerals in addition to the graphs "a" to "d". Incidentally, displaying of the time passed from the starting time of a hemodialysis treatment and a state of transitions of detected values by graphs and tables is referred to as a displaying on a time basis.

The graph "a" is obtained by plotting ΔBV values on a time basis successively detected by the ΔBV detecting means 10. The graph "b" is obtained by plotting venous blood pressures on a time basis successively detected by the hemodialysis condition detecting means 11. The graphs "c" and "d" are obtained by plotting on a time basis blood pressures and pulses successively detected by the vital sign detecting means 9. Each of the graphs is displayed in parallel on the identical screen to perceive tendencies (trends) of the current (present) detected values, thus easily comparing those detected values with each other.

According to the present carrying-out mode, since the values successively detected by the ΔBV detecting means 10 and the values successively detected by the vital sign detecting means 9 in the current (present) hemodialysis treatment can be displayed on a time basis on the identical screen, when a circulating blood volume variation rate (ΔBV) of a patient suddenly decreases during a hemodialysis treatment, the above-mentioned ΔBV can be easily compared with vital signs (such as the blood pressure and the pulse), thus easily determining whether or not the sudden decrease is an omen of shock symptoms.

In addition to the values detected by the ΔBV detecting means 10 and the values detected by the vital sign detecting means 9, since the values (venous blood pressure in the present carrying-out mode) detected by the hemodialysis condition detecting means 11 can be displayed on a time basis on the identical screen 13a, when the circulating blood volume variation rate (ΔBV) of a patient suddenly decreases, it can be easily determined whether or not the sudden decrease in the ΔBV is due to changes in the hemodialysis conditions. Note that, as long as at least the graph "a" showing ΔBV is displayed, other graphs (graphs showing the vital signs, the hemodialysis conditions and the like) may be optionally displayed.

Next, a second carrying-out mode according to the present invention will be explained.

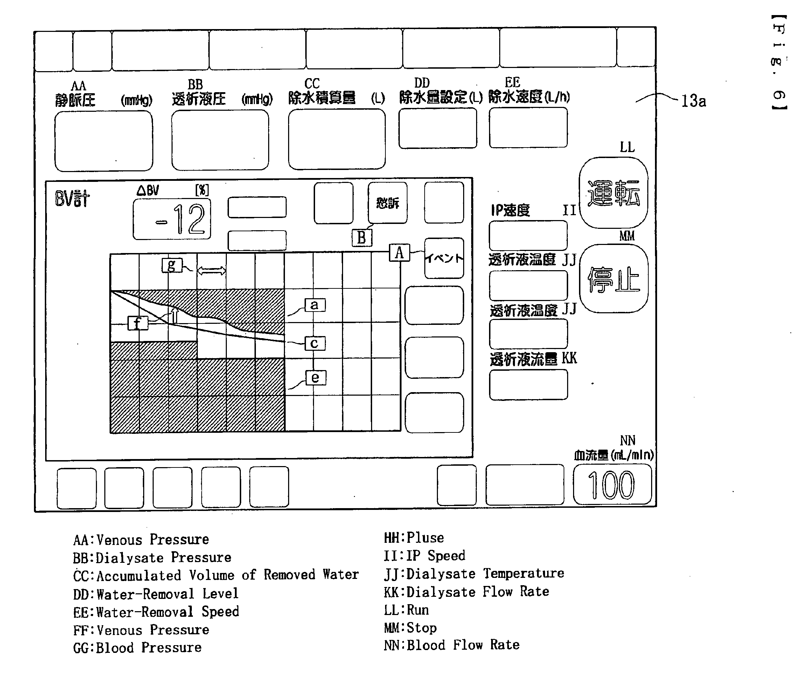

In the same way as in the aforementioned first carrying-out mode, a hemodialysis treatment apparatus according to the present carrying-out mode is used to perform hemodialysis and ultrafiltration by extracorporeally circulating blood of a patient, and is different from the first carrying-out mode only in the controlling of a display on the display means 13 by the control means 12. That is, as shown in Fig. 6, the control means 12 controls the display means 13 so that the values detected by each of the detecting means (the vital sign detecting means 9, the ΔBV detecting means 10 and the hemodialysis condition detecting means 11) are displayed on a time basis on the identical screen together with each other, and the successively detected values are displayed on the common time axis in graphs overlapping with each other.

The graph "a" is obtained by plotting ΔBV values on a time basis successively detected by the ΔBV detecting means 10. The graph "e" is obtained by plotting filtration speeds on a time basis successively detected by the hemodialysis condition detecting means 11. The graphs "c" is obtained by plotting blood pressures on a time basis successively detected by the vital sign detecting means 9. Since the graphs are displayed to overlap with each other on the identical screen, a tendency (trend) of each of the detected values can be perceived, thus easily comparing the detected values with each other.

Also, since the graphs are displayed to overlap with each other on the identical screen, a tendency (trend) of each of the detected values can be perceived and a comparison of those detected values can be visually performed, thus easily identifying a cause when a circulating blood volume variation rate (ΔBV) suddenly decreased. Note that, when each graph is displayed in colors different from each other, the graphs may be visually compared with each other more easily.

Also, the present carrying-out mode is structured to display only a graph designating a detected value arbitrarily selected from graphs being displayed and overlapping with each other. For example, when the graph "a" designating the ΔBV and the graph "c" designating the blood pressure are selected, the other graphs (in this case, the graph "e" designating the ultrafiltration speed) are not displayed. According to this structure, graphs displayed on the display means 13 are improved in the visibility, thus easily and properly comparing tendencies (trends) of required detected values with each other.

Further, the present carrying-out mode displays an operational portion A (illustrated with letters "Event") on the touch panel type screen 13a, so that touching the operational portion A with a hand can input an external factor (event) that has an influence on the hemodialysis treatment for a patient and a duration for which the external factor has influenced and displaying them on the display means 13. Such an external factor is a factor that may affect a detected ΔBV, such as a change in a body position from a lying state to a sitting-up state or a change in taking food and medicines.

In more concrete terms, as shown in Fig. 6, when there is a change in a body position of a patient, as a medical staff or the like touches the operational portion A to input the fact (there is occurred a body position change), an arrow "f" indicative the occurrence of the body position change is displayed at a predetermined point corresponding to a time of the body change in the graph "a". Also, when a continual medication and the like are performed, as the operational portion A is touched and the fact (there is occurred a medication) is inputted, an arrow "g" is displayed in the time axis direction corresponding to the duration of the medication. The arrow "g" may be displayed after touching the operational portion A at each of a start time and a completion time of medication, for example, and may be displayed after touching the operational portion A at a medication start time and an inputting of the duration, and the like. Note that, each arrow designating an external factor may be displayed with a numeral or icon for differentiating the changes in body positions, medications and the like, or may be replaced with another symbol.

Accordingly, since the external factor that has an influence on the hemodialysis treatment for a patient and the duration for the external factor has influenced can be displayed and compared with detected values displayed on a time basis, when ΔBV suddenly decreased, it is possible to easily determine whether or not the sudden decrease in ΔBV is due to external factors. Note that, the screen 13a displays an operational portion B (illustrated with letters "Complaint"), so that touching the operational portion B with a hand can input and display a degradation of physical condition complained by the patient (such as nausea and headache) thereon, in the same way as in inputting "Event".

As stated above, while the carrying-out mode of the present invention has been explained, the present invention is not restricted to the above-described carrying-out modes. The hemodialysis treatment apparatus is be provided with a storage means for storing values detected on a time basis by the ΔBV detecting means 10 in the previous or past hemodialysis treatments, and display the past detected values stored in the storage means on the identical display means 13 on a time basis as in the above-mentioned carrying-out mode, in addition to the values detected by the ΔBV detecting means 10 and the vital sign detecting means 9 in the current (present) hemodialysis treatment. In this case, a tendency of the past variation rate of circulating blood volume can be easily compared with a tendency of the current variation rate of circulating blood volume, thus performing an optimal hemodialysis treatment.

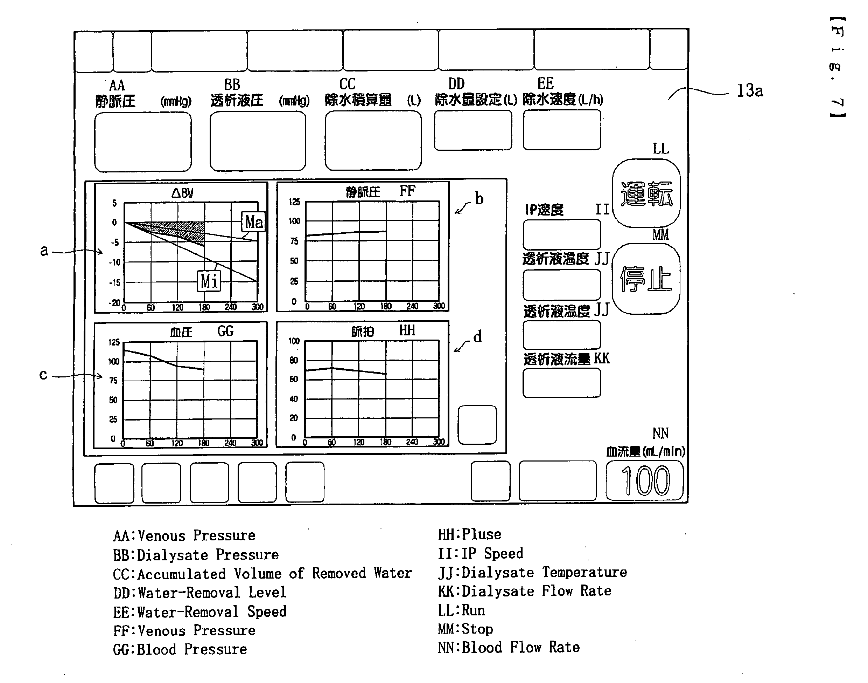

Specifically, it is preferable to obtain an ideal range of transitions of values detected by the ΔBV detecting means 10 based on the past detected values stored in the storage means, and as shown in a graph "a" in Fig. 7, display an upper limited value Ma and a lower limited value Mi of the ideal range of transitions on a time basis on the identical screen. Such an ideal transition of ΔBV is determined by a medical staff such as a doctor by referring to the past transitions of ΔBV, the past complains, presence or absence of treatments, and results of hemodialysis (optimality of values relating to ultrafiltration and blood tests).

Accordingly, when an ideal range of transition of detected values is obtained based on the past values detected by the ΔBV detecting means 10 and the upper limited value and the lower limited value of the ideal range of transition are displayed on a time basis on the identical screen 13a, it is possible to monitor in real-time whether or not the current value detected by the ΔBV detecting means 10 is within the ideal range of transition, and to easily compare the current value with the value detected by the vital sign detecting means 9 when the current value deviates from the ideal range of transition.

Note that, when the past detected values are displayed, only predicted values or optimal values of ΔBV can be displayed on the identical screen 13a, in addition to graphs. Also, when the ideal range of transition of ΔBV is displayed as explained above, the ideal range may be displayed as a band graph having a width from the upper limit to the lower limit. In addition, a regression curve (single curve different from a pair of graphs having the upper limit and the lower limit) may be obtained based on transitions of a plurality of the past ΔBV's, and displayed on the identical screen 13a.

Moreover, according to the present carrying-out mode, ΔBV is calculated and detected based on the hematocrit value Ht detected by the hematocrit sensor 5, however, ΔBV may be detected by using a calculation or a prediction based on other parameters. In addition, according to the present carrying-out mode, the blood pressure and the pulse of a patient are detected by the vital sign detecting means 9, however, other parameters may be detected as long as the parameters are vital signs of the patient under treatment.

Furthermore, according to the present carrying-out mode, each of the successively detected values is displayed on the screen 13a of the display means 13 in graphs, however, the successively detected values may be displayed in tables or the like on a time basis on the identical screen. Note that, each of the lengths of the time axes on the screen 13a of the display means 13 may be set to a fixed length (when the duration of a hemodialysis treatment is longer than a predetermined time, the time axis is reduced, and when shorter, the time axis is extended) so that the duration of time in relation to the duration of the hemodialysis treatment may be standardized and displayed. Although the display means 13 is of a touch panel type, the display means 13 may be structured by other screen (such as a non-touch-panel type of liquid crystal screen).

### INDUSTRIAL POTENTIAL OF UTILIZATION

The present invention may be applied to other modes of hemodialysis treatment apparatuses which has a different outer shape or is provided with other additional functions, as long as such hemodialysis treatment apparatuses are provided with a display means capable of displaying values detected by a circulating blood volume variation rate detecting means and values detected by a vital sign detecting means on a time basis on the identical screen.

### BRIEF DESCRIPTION OF THE DRAWINGS

- [Fig. 1]: A schematic diagram illustrating a structure of the hemodialysis treatment apparatus according to a first carrying-out mode of the present invention.
- [Fig. 2]: A schematic diagram illustrating an internal structure of a dialysis device body according to the first carrying-out mode of the present invention.
- [Fig. 3]: A perspective view illustrating an external appearance of the dialysis device body according to the first carrying-out mode of the present invention.
- [Fig. 4]: A block diagram illustrating the internal structure of the dialysis device body according to the first carrying-out mode of the present invention
- [Fig. 5]: A schematic diagram illustrating a screen of the dialysis device body according to the first carrying-out mode of the present invention.
- [Fig. 6]: A schematic diagram illustrating a screen displayed by a display means of the dialysis device body of the hemodialysis treatment apparatus according to a second carrying-out mode of the present invention.
- [Fig. 7]: A schematic diagram illustrating a screen displayed by a display means according to another carrying-out mode of the present invention.

## Claims

1. A hemodialysis treatment apparatus for carrying out a hemodialysis treatment by performing hemodialysis and ultrafiltration upon an extracorporeally-circulating blood of a patient, **characterized by** comprising:
a circulating blood volume variation rate detecting means (10) for successively detecting a variation rate of a volume of circulating blood of said patient as a hemodialysis treatment time passes;
a vital sign detecting means (9) for successively detecting a vital sign of said patient as a hemodialysis treatment time passes;
a display means (13) capable of displaying a value detected by said circulating blood volume variation rate detecting means (10) and a value detected by said vital sign detecting means (9) on a time basis on an identical screen, and
a storage means for storing values detected on a time basis by said circulating blood volume variation rate detecting means (10) in past hemodialysis treatments, **characterized in that** said display means (13) is configured to display past detected values stored in said storage means on said identical screen (13a) on a time basis in addition to the values detected by said circulating blood volume variation rate detecting means (10) and said vital sign detecting means (9) in said hemodialysis treatment.

2. The hemodialysis treatment apparatus as set forth in claim 1, **characterized by** a hemodialysis condition detecting means (11) for successively detecting a hemodialysis condition relating to hemodialysis and ultrafiltration as the hemodialysis treatment time passes, said display means (13) being configured to display a value detected by said hemodialysis condition detecting means (11) in addition to the value detected by said circulating blood volume variation rate detecting means (10) and the value detected by said vital sign detecting means (9) on a time basis on said identical screen (13a).

3. The hemodialysis treatment apparatus as set forth in claim 1 or claim 2, **characterized in that** said display means (13) is configured to display each of successively-detected values in graphs each with an abscissa designating the time.

4. The hemodialysis treatment apparatus as set forth in claim 3, **characterized in that** said display means (13) is configured to display each of said successively-detected values in a plurality of graphs overlapping with each other and with a common time axis.

5. The hemodialysis treatment apparatus as set forth in claim 4, **characterized in that** said display means (13) is configured to display only a graph designating a detected value arbitrarily selected among said graphs being displayed and overlapping with each other.

6. The hemodialysis treatment apparatus as set forth in any one of claim 1 to claim 5, **characterized in that** said display means is configured to display (13) an external factor that has an influence on said hemodialysis treatment for said patient and a duration where said external factor has influenced, so that said detected values are compared with each other on a time basis.

7. The hemodialysis treatment apparatus as set forth in claim 1, **characterized in that** an ideal range of transitions is obtained for said values detected by said circulating blood volume variation rate detecting means (10) based on said past detected values stored in said storage means, so that an upper limited value and a lower limited value of said ideal range of transitions are displayed on a time basis on said identical screen (13a).

## Patentansprüche

1. Hämodialyse-Behandlungsvorrichtung zum Ausführen einer Hämodialyse-Behandlung mittels Durchführung einer Hämodialyse und einer Ultrafiltration bei einem extrakorporal zirkulierenden Blut eines Patienten, **dadurch gekennzeichnet, dass** sie aufweist:
eine Einrichtung (10) zum Erfassen einer Änderungsrate eines zirkulierenden Blutvolumens, um sukzessive eine Änderungsrate eines Volumens eines zirkulierenden Blutes des Patienten im Verlauf einer Hämodialyse-Behandlungszeit zu erfassen;
eine Vitalzeichen-Erfassungseinrichtung (9), um sukzessive ein Vitalzeichen des Patienten während des Verlaufs der Hämodialyse-Behandlungszeit zu erfassen; und
eine Anzeigeeinrichtung (13), die fähig ist, einen Wert anzuzeigen, der durch die Einrichtung (10) zur Erfassung der Änderungsrate eines zirkulierenden Blutvolumens erfasst wird, und einen Wert anzuzeigen, der durch die Vitalzeichen-Erfassungseinrichtung (9) erfasst wird, und zwar auf einer Zeitbasis auf einem identischen Bildschirm, und
eine Speichereinrichtung, um Werte zu speichern, die auf einer Zeitbasis durch die Einrichtung (10) zur Erfassung einer Änderungsrate eines zirkulierenden Blutvolumens bei früheren Hämodialyse-Behandlungen erfasst wurden, **dadurch gekennzeichnet, dass** die Anzeigeeinrichtung (13) konfiguriert ist, um die früher erfassten Werte, die in der Speichereinrichtung gespeichert sind, auf dem identischen Bildschirm (13a) auf einer Zeitbasis anzuzeigen, zusätzlich zu den Werten, die durch die Einrichtung (10) zur Erfassung einer Änderungsrate eines zirkulierenden Blutvolumens und der Einrichtung (9) zur Erfassung eines Vitalzeichens bei der Hämodialyse-Behandlung erfasst werden.

2. Hämodialyse-Behandlungsvorrichtung nach Anspruch 1, **gekennzeichnet durch** eine Hämodialysezustands-Erfassungseinrichtung (11), um sukzessive einen Hämodialysezustand betreffend eine Hämodialyse und eine Ultrafiltration im Verlauf der Hämodialyse-Behandlungszeit zu erfassen, wobei die Anzeigeeinrichtung konfiguriert ist, um einen Wert anzuzeigen, der **durch** die Hämodialysezustands-Erfassungseinrichtung (11) erfasst wird, zusätzlich zu dem Wert, der **durch** die Einrichtung (10) zur Erfassung einer Änderungsrate eines zirkulierenden Blutvolumens erfasst wird, und dem Wert, der **durch** der Einrichtung (9) zur Erfassung eines Vitalzeichens erfasst wird, und zwar auf einer Zeitbasis auf dem identischen Bildschirm (13a)

3. Hämodialyse-Behandlungsvorrichtung nach Anspruch 1 oder 2, **dadurch gekennzeichnet, dass** die Anzeigeeinrichtung (13) konfiguriert ist, um einen jeweiligen von sukzessive erfassten Werten in Graphen jeweils mit einer die Zeit bezeichnenden Abszisse anzuzeigen.

4. Hämodialyse-Behandlungsvorrichtung nach Anspruch 3, **dadurch gekennzeichnet, dass** die Anzeigeeinrichtung (13) konfiguriert ist, um einen jeweiligen der sukzessive erfassten Werten in einer Mehrzahl von Graphen, die einander überlappen, und mit einer gemeinsamen Zeitachse anzuzeigen.

5. Hämodialyse-Behandlungsvorrichtung nach Anspruch 4, **dadurch gekennzeichnet, dass** die Anzeigeeinrichtung (13) konfiguriert ist, um lediglich einen Graph anzuzeigen, der einen erfassten Wert bezeichnet, welcher willkürlich aus den Graphen ausgewählt wird, die angezeigt werden und einander überlappen.

6. Hämodialyse-Behandlungsvorrichtung nach einem der Ansprüche 1 bis 5, **dadurch gekennzeichnet, dass** die Anzeigeeinrichtung (13) konfiguriert ist, um einen externen Faktor, welcher einen Einfluss auf die Hämodialyse-Behandlung für den Patienten hat, und eine Dauer anzuzeigen, bei der dieser externe Faktor beeinflusst hat, so dass die erfassten Werte miteinander auf einer Zeitbasis verglichen werden.

7. Hämodialyse-Behandlungsvorrichtung nach Anspruch 1, **dadurch gekennzeichnet, dass** ein idealer Bereich von Übergängen für die Werte erzielt wird, die durch die Einrichtung (10) zur Erfassung einer Änderungsrate eines zirkulierenden Blutvolumens erfasst werden, und zwar basierend auf den früher erfassten Werten, die in der Speichereinrichtung gespeichert sind, so dass ein oberer Grenzwert und ein unterer Grenzwert des idealen Bereiches von Übergängen auf einer Zeitbasis auf dem identischen Bildschirm (13a) angezeigt werden.

## Revendications

1. Appareil de traitement d'hémodialyse, pour effectuer un traitement d'hémodialyse en accomplissant une hémodialyse et une ultrafiltration dans le cadre d'une circulation extracorporelle du sang d'un patient, **caractérisé par** le fait de comprendre :
des moyens de détection du taux de variation de volume sanguin en circulation (10), pour détecter successivement un taux de variation d'un volume de sang en circulation dudit patient, au fur et à mesure de l'écoulement du temps de traitement par hémodialyse ;
des moyens de détection de signe vital (9), pour détecter successivement un signe vital dudit patient, au fur et à mesure de l'écoulement du temps de traitement par hémodialyse ;
des moyens d'affichage (13), capables d'afficher une valeur détectée par lesdits moyens de détection du taux de variation de volume sanguin en circulation (10) et une valeur détectée par lesdits moyens de détection de signe vital (9), sur une base de temps, sur un écran identique, et
des moyens de stockage, pour stocker des valeurs détectées, sur une base de temps, par lesdits moyens de détection du taux de variation de volume sanguin en circulation (10), lors de traitements par dialyse passés, **caractérisé en ce que** lesdits moyens d'affichage (13) sont configurés pour afficher des valeurs passées détectées, stockées dans lesdits moyens de stockage, sur ledit écran identique (13a), sur une base de temps, en plus des valeurs
détectées par lesdits moyens de détection du taux de variation de volume sanguin en circulation (10) et lesdits moyens de détection de signe vital (9), dans ledit traitement par hémodialyse.

2. Appareil de traitement d'hémodialyse selon la revendication 1, **caractérisé par** des moyens de détection de condition d'hémodialyse (11), pour successivement détecter une condition d'hémodialyse concernant une hémodialyse et une ultrafiltration, au fur et à mesure de l'écoulement du temps de traitement par hémodialyse, lesdits moyens d'affichage (13) étant tels qu'ils affichent une valeur détectée par lesdits moyens de détection de condition d'hémodialyse (11), en plus de la valeur détectée par lesdits moyens de détection du taux de variation de volume sanguin en circulation (10) et de la valeur détectée par lesdits moyens de détection de signe vital (9), sur une base de temps, sur ledit écran identique (13a).

3. Appareil de traitement d'hémodialyse selon la revendication 1 ou la revendication 2, **caractérisé en ce que** lesdits moyens d'affichage (13) sont configurés pour afficher chacune des valeurs détectées successivement, dans des graphiques, chacun avec une abscisse désignant le temps.

4. Appareil de traitement d'hémodialyse selon la revendication 3, **caractérisé en ce que** lesdits moyens d'affichage (13) sont configurés pour afficher chacune desdites valeurs détectées successivement, dans une pluralité de graphiques, se chevauchant les uns les autres et avec un axe des temps commun.

5. Appareil de traitement d'hémodialyse selon la revendication 4, **caractérisé en ce que** lesdits moyens d'affichage (13) sont configurés pour afficher uniquement un graphique désignant une valeur détectée, sélectionné arbitrairement parmi lesdits graphiques affichés et se chevauchant les uns les autres.

6. Appareil de traitement d'hémodialyse selon l'une quelconque des revendications 1 à 5, **caractérisé en ce que** lesdits moyens d'affichage (13) sont configurés pour afficher un facteur externe, ayant une influence sur ledit traitement par hémodialyse pour ledit patient, et une durée pendant laquelle ledit facteur externe a exercé son influence, de manière que lesdites valeurs détectées soient comparées entre elles sur une base de temps.

7. Appareil de traitement d'hémodialyse selon la revendication 1, **caractérisé en ce qu'**une plage idéale de transitions est obtenue pour lesdites valeurs détectées par lesdits moyens de détection du taux de variation de volume sanguin en circulation (10), d'après des valeurs passées détectées, stockées dans lesdits moyens de stockage, de manière qu'une valeur de limite supérieure et une valeur de limite inférieure de ladite plage idéale de transitions soient affichées sur une base de temps, sur ledit écran identique (13a).
